# EUROPEAN PATENT APPLICATION

(11) **EP 4 275 594 A1**
(43) Date of publication of application: **15.11.2023**
(21) Application number: 21917880.3
(22) Date of filing: 24.11.2021
(51) Int. Cl.: A61B 5/087, A61B 5/091, A63B 23/18

(54) **RESPIRATION ADJUSTMENT DEVICE AND RESPIRATION MEASUREMENT MOUTHPIECE ASSEMBLY INCLUDING SAME**

(30) Priority: 07.01.2021 KR 20210001728
(71) Applicant: Breathings Co., Ltd., Gangwon-do 26354 (KR)
(72) Inventor: LEE, In Pyo, Seoul 06249 (KR); YOON, Ki Sang, Yongin-si Gyeonggi-do 17002 (KR); SONG, Chang Ho, Seongnam-si Gyeonggi-do 13584 (KR)
(74) Representative: dompatent von Kreisler Selting Werner - Partnerschaft von Patent- und Rechtsanwälten mbB
(86) International application number: PCT/KR2021/017350
(87) International publication number: WO 2022/149718

(57) **Abstract**

The present invention may provide a respiration adjustment device and a respiration measurement mouthpiece assembly including same, the device comprising: a body part in which an inlet part through which air exhaled by breathing of a user is introduced, an outlet part through which air is discharged and which is coupled to a mouthpiece, and an internal flow path are formed; and a pressure regulator provided in the internal flow path and adjusting the pressure of air through the manipulation of the user. According to the description above, a user can adjust respiratory pressure by himself or herself according to the user's health condition and thus perform a breathing exercise suitable for each individual user. Therefore, the present invention can increase an effect of the breathing exercise.

## Description

### [Technical Field]

The present invention relates to a respiration control device and a mouthpiece assembly for respiration measurement including the same, and more particularly to a respiration control device and a mouthpiece assembly for respiration measurement including the same, configured to allow a user not only to measure his or her respiration but also to perform respiration exercise during respiration measurement and to provide respiration pressure suitable for the health state of the user, thereby increasing an effect of respiration exercise.

### [Background Art]

In general, a respiration measurement device is a device configured to measure and analyze respiration volume and lung capacity of a user, and is an essential tool for lung health management. The respiration measurement device may include a respiration tube, that is, a mouthpiece placed in the mouth of a user and configured to allow the user to breathe in and out therethrough in order to analyze respiration volume of the user.

Meanwhile, as an example of a technique for the respiration measurement device including such a mouthpiece, Korean Patent No. 10-1070960 discloses a portable spirometry device including a housing, a straight pipe formed to penetrate the inside of the housing, a rotating body disposed on the straight pipe and configured to rotate according to the flow of air, a light emitting diode and a light receiving diode exposed to the pipe with the rotating body interposed therebetween so that light emitted by rotation of the rotating body is intermittently received, a controller configured to check a state change in pulse applied from the light receiving diode and to calculate the rate of rotations of the rotating body and pulses per second, and a display mounted on one side of the housing so as to display lung capacity with a digital value through the calculated value of the controller.

However, the above-described conventional respiration measurement device is configured to measure only a respiration volume. Therefore, when user's respiration is measured, the conventional respiration measurement device may not provide a user with another function and effect such as respiration exercise in addition to respiration measurement.

### [Disclosure]

### [Technical Problem]

Therefore, the present invention has been made in view of the above problems, and it is an object of the present invention to provide a respiration control device and a mouthpiece assembly for respiration measurement including the same, configured to allow a user not only to measure his or her respiration but also to perform respiration exercise during respiration measurement and to allow a user to directly control respiration pressure depending on the user's health state, thereby making it possible not only to perform respiration exercise suitable for the health state of each user but also to increase an effect of respiration exercise.

### [Technical Solution]

In accordance with an aspect of the present invention, the above and other objects can be accomplished by the provision of a respiration control device coupled to a mouthpiece of a respiration measurement device and configured to control respiration pressure of a user during user's respiration, the respiration control device including a body part including an inflow part, an outflow part, and an internal flow path, wherein the inflow part is formed on one side of the body part and configured to allow air by the user's respiration to flow thereinto, wherein the outflow part is formed on the other side of the body part, configured to allow the air flowing into the inflow part to flow out therethrough, and coupled to the mouthpiece, and wherein the internal flow path is formed in the body part and configured to allow the inflow part and the outflow part to communicate with each other, and a pressure controller provided on the internal flow path and configured to control pressure of the air flowing through the internal flow path by an operation of the user, wherein the pressure controller includes a moving member provided on the internal flow path and configured to reciprocate thereon, wherein the moving member adjusts an air passage cross-section area of the internal flow path by reciprocation thereof and controls the pressure of the air, a driving part rotatably installed in the body part, coupled to the moving member, and configured to allow the moving member to reciprocate by rotation thereof, and a rotation lever rotated by the operation of the user and axially coupled to the driving part, wherein the rotation lever rotates the driving part by rotation thereof.

In accordance with another aspect of the present invention, there is provided a mouthpiece assembly including the respiration control device and a mouthpiece detachably coupled to a main body including a respiration measurement module, wherein the mouthpiece has an air flow path formed therein and is coupled to the respiration control device.

### [Advantageous effects]

A respiration control device and a mouthpiece assembly for respiration measurement including the same according to the present invention are detachably coupled to a mouthpiece of a respiration measurement device so as to perform respiration measurement and respiration exercise at the same time, thereby enabling a user to perform respiration exercise. Further, the respiration control device and the mouthpiece assembly are configured to allow a user to directly control respiration pressure depending on the user's health state, thereby making it possible not only to perform respiration exercise suitable for the health state of each user but also to increase an effect of respiration exercise.

Further, in the respiration control device and the mouthpiece assembly for respiration measurement including the same according to the present invention, since respiration pressure is controllable by a simple user operation, it is easy to use. Additionally, since the structure is simple and manufacturing is easy, an economic effect may be achieved.

In the respiration control device and the mouthpiece assembly for respiration measurement including the same according to the present invention, the respiration control device may be selectively and easily coupled to a mouthpiece as necessary, and may be easily detachable from the mouthpiece. Accordingly, the respiration control device and the mouthpiece assembly are easy to carry and store.

### [Description of Drawings]

The above and other objects, features and other advantages of the present invention will be more clearly understood from the following detailed description taken in conjunction with the accompanying drawings, in which:
FIG. 1 is a perspective view showing a respiration control device and a mouthpiece assembly including the same according to an embodiment of the present invention;
FIG. 2 is an exploded perspective view showing a case in which the respiration control device and a mouthpiece are separated from the mouthpiece assembly in FIG. 1;
FIG. 3 is an exploded perspective view showing the internal configuration of the respiration control device in FIG. 1;
FIG. 4 is a cross-sectional view showing a respiration control example of the respiration control device in FIG. 1;
FIG. 5 is a perspective view showing the configuration and operation of a recognition means provided in the respiration control device in FIG. 1;
FIG. 6 is a perspective view showing a respiration control device and a mouthpiece assembly including the same according to another embodiment of the present invention;
FIG. 7 is an exploded perspective view showing a case in which the respiration control device and a mouthpiece are separated from the mouthpiece assembly in FIG. 6;
FIG. 8 is a perspective view showing a respiration control example of the respiration control device in FIG. 6; and
FIG. 9 is a perspective view showing the configuration and operation of a recognition means provided in the respiration control device in FIG. 6.

### [Best Mode]

Hereinafter, preferred embodiments of the present invention will be described with reference to the accompanying drawings so as to explain the present invention in more detail.

Since the present invention may have various modifications and embodiments, specific embodiments are illustrated in the drawings and described in detail. However, it is not intended to limit the present invention to the specific embodiments, and it should be understood that the specific embodiments cover all modifications, equivalents, and substitutes within the spirit and technical scope of the present invention.

Terms such as "first", "second", and the like may be used herein to describe various components, but the components should not be limited by the terms. The terms are used only for the purpose of distinguishing one component from another component. For example, a first component may be referred to as a second component and, similarly, a second component may be referred to as a first component without departing from the scope of the present invention. Terms such as "and/or" include a combination of a plurality of related described items or any one of a plurality of related described items.

It will be understood that, when an element is referred to as being "connected" or "joined" to another element, the element may be directly connected or joined to the other element, but it should be understood that any intervening element may also be present therebetween. In contrast, when an element is referred to as being "directly connected" or "directly joined" to another element, it should be understood that no intervening element is present therebetween.

The terms used in the present application are used only to describe specific embodiments and are not intended to limit the present invention. Singular forms are intended to include plural forms as well, unless the context clearly indicates otherwise. It will be further understood that the terms "comprises", "includes", and/or "has", when used in the present application, specify the presence of stated features, integers, steps, operations, elements, components, or combinations thereof, but do not preclude the presence or addition of one or more other features, integers, steps, operations, elements, components, or combinations thereof.

Unless defined otherwise, all terms used herein, including technical or scientific terms, have the same meaning as those generally understood by those skilled in the art to which the present invention pertains. Terms such as those defined in commonly used dictionaries should be interpreted as having meanings consistent with those in the context of the related art. Unless explicitly defined in the present application, the terms are not interpreted in an ideal or overly formal sense. In addition, in order to facilitate the overall understanding in describing the present invention, the same components in the drawings are denoted by the same reference numerals, and duplicate descriptions of the same components are omitted.

Hereinafter, preferred embodiments of the present invention will be described in more detail with reference to the accompanying drawings.

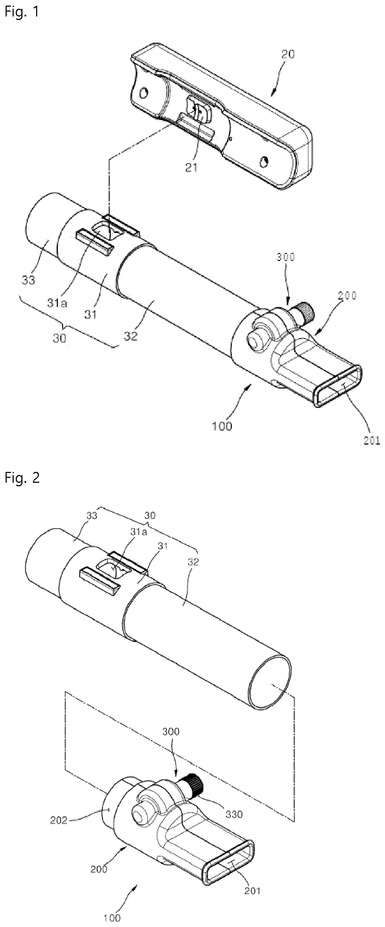

Referring to FIG. 1, a respiration control device 100 according to an embodiment of the present invention may be coupled to a mouthpiece 30 of a respiration measurement device (not shown) so as to control user's respiration pressure during respiration.

Meanwhile, prior to description of the respiration control device 100, a description will be given as to the mouthpiece 30 having the respiration control device 100 detachably coupled thereto.

The mouthpiece 30 has a configuration in which a user directly puts his or her mouth on the respiration measurement device and breathes in and out therethrough. The mouthpiece 30 may include a tubular tube adapter 31, a first tube 32 detachably fitted to one side of the tube adapter 31 in a tubular shape, and a second tube 33 detachably fitted to the other side of the tube adapter 31 in a tubular shape.

In detail, the tube adapter 31 is detachably coupled to a main body 20 having a respiration measurement module 21 provided on the outer circumferential side surface thereof, the main body 20 having a cylindrical tube shape, and the same has an insertion part 31a provided on the outer circumferential side surface thereof and configured to allow the respiration measurement module 21 to be inserted thereinto so as to be sealed when coupled to the main body 20.

Here, the respiration measurement module 21 includes a differential pressure generation part formed to protrude from a side surface thereof, the side surface facing an internal air flow path, so as to have a predetermined thickness and a predetermined height and configured to generate differential pressure. The respiration measurement module 21 has an inlet part and an outlet part respectively formed on the opposite sides of the differential pressure generation part, and the same may be configured to detect differential pressure between the inlet part and the outlet part generated by the differential pressure generation part.

In this case, the respiration measurement module 21 may have a sensing flow path formed therein and configured to communicate with the inlet part and the outlet part, and a sensing unit (not shown) configured to detect a pressure difference between the inlet part and the outlet part, but is not limited thereto. The respiration measurement module 21 may include an air pressure sensor or a pressure sensor capable of detecting a pressure difference between the opposite sides of the differential pressure generation part.

Referring to FIG. 2, the first tube 32 with a cylindrical tube shape may have one end detachably coupled to one side of the tube adapter 31, and the other end having the respiration control device 100 detachably coupled thereto. In this case, the first tube 32 has an outer circumferential surface of the one end, the outer circumferential surface contacting the inner circumferential surface of the tube adapter 31 so as to be fitted thereto, and an inner circumferential surface of the other end, the inner circumferential surface having the respiration control device 100 insertable thereinto and couplable thereto.

The second tube 33 with a cylindrical tube shape may have one end detachably coupled to the other side of the tube adapter 31, and the outer circumferential surface of the one end may contact the inner circumferential surface of the tube adapter 31 so as to be fitted thereto.

Meanwhile, in the drawing, the respiration control device 100 may be detachably coupled to the exposed end of the first tube 32, and the mouthpiece 30 may be configured to supply air by user's respiration to the first tube 32 when a user puts his or her mouth on the respiration control device 100 and breathes in and out therethrough. Here, it goes without saying that the respiration control device 100 may be coupled to the second tube 33 in the embodiment.

Hereinafter, the respiration control device 100 will be described in detail.

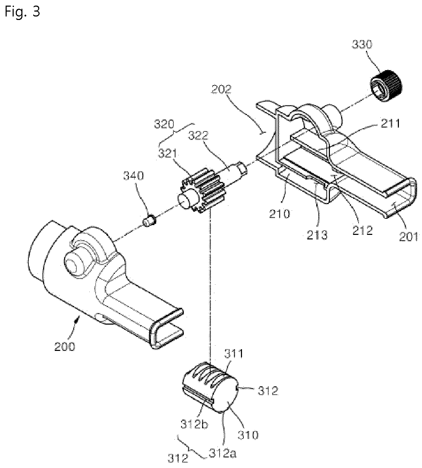

Referring to FIG. 3, the respiration control device 100 may include a body part 200 and a pressure controller 300.

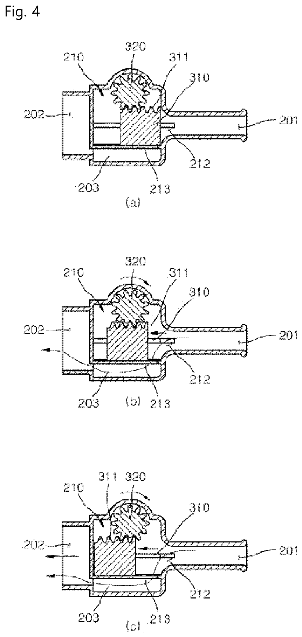

The body part 200 is detachably coupled to the mouthpiece 30, and when a user directly puts his or her mouth on the respiration control device 100 to breathe in and out, air by user's respiration may flow into the mouthpiece 30 through an internal flow path 203 (refer to FIG. 4).

In detail, the body part 200 may have an inflow part 201 formed on one side on which a user puts his or her mouth and configured to allow air by user's respiration to flow thereinto, an outflow part 202 formed on the other side coupled to the mouthpiece 30 and configured to allow the air flowing thereinto to flow out therethrough, and the internal flow path 203 formed therein and configured to allow the inflow part 201 and the outflow part 202 to communicate with each other.

Here, the inflow part 201 communicates with an inlet part 212 of a control room 210, and as shown in the drawing, the same may be configured to form a long and narrow flow path corresponding to the size of the user's mouth. In the drawing, the inflow part 201 is formed in a square duct tube shape to correspond to the shape and size of the user's mouth, so that the user may more easily breathe in and out while holding the inflow part 201 in the mouth. However, the above-described shape of the inflow part 201 is a preferred embodiment, and it goes without saying that the inflow part 201 may be formed in various shapes such as an elliptical tube shape in addition to the square duct tube shape.

The body part 200 has the pressure controller 300 formed on the internal flow path 203, and the control room 210 formed therein and configured to control pressure of air flowing by the operation of the pressure controller 300.

The body part 200 may include a partition wall 211 formed therein and configured to support a moving member 310 and to form the control room 210. Here, in the control room 210, a space is formed in the body part 200 by the partition wall 211, and the pressure controller 300 may be installed in the space. Further, the control room 210 may have the inlet part 212 formed therein and configured to communicate with the inflow part 201 so as to allow air to flow thereinto, and an outlet part 213 formed therein and configured to allow the introduced air to flow out therethrough after pressure of the introduced air is controlled through the pressure controller 300.

Meanwhile, in the above description, the partition wall 211 supports the pressure controller 300 so that the pressure controller 300 is located in the set position and operated therein, and the same may be configured in various forms within the inner space so as to form the inlet part 212 and the outlet part 213.

In the drawing, the partition wall 211 includes a lower wall with a plate shape disposed in the horizontal direction corresponding to the movement direction of the moving member 310 and located below the moving member 310 to support the moving member 310 upwards, the lower wall having the outlet part 213 formed thereon, a side wall located on the side of the moving member 310 and configured to guide the moving member 310, and a rear wall built behind the moving member 310.

Here, the partition wall 211 may be formed to limit rearward movement of the moving member 310 through the rear wall so as to limit sliding movement of the moving member 310, and to limit forward movement of the moving member 310 by a locking jaw formed by causing the front end of the lower wall to be located lower than the upper surface of the inflow part 201.

Meanwhile, the moving member 310 may adjust an air passage cross-sectional area of each of the inlet part 212 and the outlet part 213 by movement thereof in the control room 210, and through this movement, pressure of flowing air is controlled. Here, pressure control in the control room 210 will be described in more detail in the operation of the pressure controller 300 in FIG. 4 to be described later.

Hereinafter, the pressure controller 300 will be described.

The pressure controller 300 is provided in the control room 210 on the internal flow path 203, and the same is operated by user's operation to perform a function of controlling pressure of air flowing through the internal flow path 203.

Specifically, the pressure controller 300 may include the moving member 310, a driving part 320, and a rotation lever 330.

First, the moving member 310 is provided on the internal flow path 203 and moves forwards and rearwards. Further, the moving member 310 may be configured to adjust an air passage area of the internal flow path 203 by movement thereof, thereby controlling air pressure.

In detail, the moving member 310 is installed in the control room 210 and the surface thereof slides along the side surface of the partition wall 211. Further, the moving member 310 may be configured to adjust, by movement thereof, an air passage cross-sectional area of each of the inlet part 212 and the outlet part 213 of the control room 210.

In the drawings, a description has been given as to a case in which the moving member 310 is formed in a substantially cylindrical shape, and the upper and lower outer circumferential side surfaces thereof are formed to slide along the partition wall 211, but the present invention is not limited to the case.

Meanwhile, the moving member 310 may have a guide protrusion formed on the lower outer circumferential side surface thereof in the axial direction and inserted into a sliding groove formed on an lower wall of the partition wall 211 so as to slide along the sliding groove.

In addition, the moving member 310 may have a plurality of moving grooves 311 continuously formed on the upper outer circumferential side surface thereof in the movement direction, that is, in the axial direction. Here, the moving grooves 311 are coupled to the driving part 320 to allow the moving member 310 to be moved by rotation of the driving part 320.

The moving member 310 may have a guide portion 312 formed on the outer surface thereof and configured to move along a fitting portion of the partition wall 211 to be described later. Here, one or more guide portions 312 may be formed in the axial (length) direction from one end of the outer surface of the moving member 310 to the other end thereof.

As shown in the drawing, the guide portion 312 may have a guide protrusion 312a formed thereon and a guide groove 312b formed therein. The guide protrusion 312a and the guide groove 312b may be formed to be spaced apart from each other on the outer circumferential side surface of the moving member 310 in the circumferential direction.

Meanwhile, the partition wall 211 facing the moving member 310 has fitting portions formed at positions respectively corresponding to the guide protrusion 312a and the guide groove 312b, so that the guide protrusion 312a and the guide groove 312b are respectively fitted to the fitting portions and coupled thereto, thereby performing guidance of the moving member 310. As shown in the drawing, regarding the fitting portions, the side wall of the partition wall 211 may be formed in a rib shape so as to be fitted into the guide groove 312b, and the partition wall 211 may have a fitting groove formed in the lower wall thereof and configured to allow the guide protrusion 312a to be fitted thereinto.

The driving part 320 is rotatably installed in the body part 200, and the same is coupled to the moving member 310 to enable the moving member 310 to reciprocate in a straight line by rotation thereof.

Specifically, the driving part 320 may include a rotation shaft 322 and a driving gear 321.

The rotation shaft 322 may be axially coupled to the rotation lever 330 to rotate in conjunction with rotation of the rotation lever 330.

The driving gear 321 is axially coupled to the rotation shaft 322 and rotates in conjunction with rotation of the rotation shaft 322, and the same has a screw thread formed on the outer circumferential surface thereof and engaged with the moving groove 311, thereby allowing the moving member 310 to reciprocate according to rotation of the driving gear 321.

The rotation lever 330 is provided on the outside of the body part 200 and is rotated by user's operation. Further, the rotation lever 330 is axially coupled to the rotation shaft 322 of the driving part 320. Accordingly, when a user operates the rotation lever 330 to rotate the same, the rotation shaft 322 may be rotated in conjunction with rotation of the rotation lever 330.

Hereinafter, the operation of the respiration control device 100 according to the operation of the pressure controller 300 will be described with reference to FIG. 4.

First, FIG. 4(a) shows a case in which respiration pressure is maximum, FIG. 4(b) shows a case in which respiration pressure is medium, and FIG. 4 (c) shows a case in which respiration pressure is minimum.

First, referring to FIG. 4(a), the moving member 310 of the pressure controller 300 is moved as close as possible to the inlet part 212 (right side in the drawing). Accordingly, the inlet part 212 and the outlet part 213 are shielded by the moving member 310 of the pressure controller 300 so that respiration pressure is maximized.

Next, referring to FIG. 4 (b), when a user operates the rotation lever 330 of the pressure controller 300 to rotate the driving part 320, as shown in the drawing, the moving member 310 is moved (left side in the drawing) to be spaced apart from the inlet part 212 by a predetermined distance. In this case, the inlet part 212 and the outlet part 213 are partially opened by the pressure controller 300, and an air passage area is increased as compared with the case in FIG. 4(a), which results in lower respiration pressure.

Finally, referring to FIG. 4(c), when a user additionally operates the rotation lever 330 of the pressure controller 300 to further rotate the driving part 320, as shown in the drawing, the moving member 310 is moved (left side in the drawing) to be maximally spaced apart from the inlet part 212. In this case, both the inlet part 212 and the outlet part 213 are opened by the pressure controller 300, and an air passage area is maximized, which results in minimum respiration pressure. Accordingly, a user may breathe most comfortably.

According to the above description, in the pressure controller 300, when air is introduced from the inlet part 212 at the front end in the movement direction of the moving member 310, the introduced air flows out through the outlet part 213 formed on the side of the moving member 310, and an air passage area of each of the inlet part 212 and the outlet part 213 may be adjusted by movement of the moving member 310, thereby controlling respiration pressure. That is, in the pressure controller 300 of the present invention, a user may freely and directly control respiration pressure by rotating the rotation lever 330 depending on the health state of the user, thereby making it possible not only to provide respiration exercise suitable for the individual health state, but also to increase an effect of respiration exercise.

Meanwhile, the pressure controller 300 may further include a recognition means 340 provided on the body part 200 and configured to allow a user to recognize whether the pressure controller 300 operates.

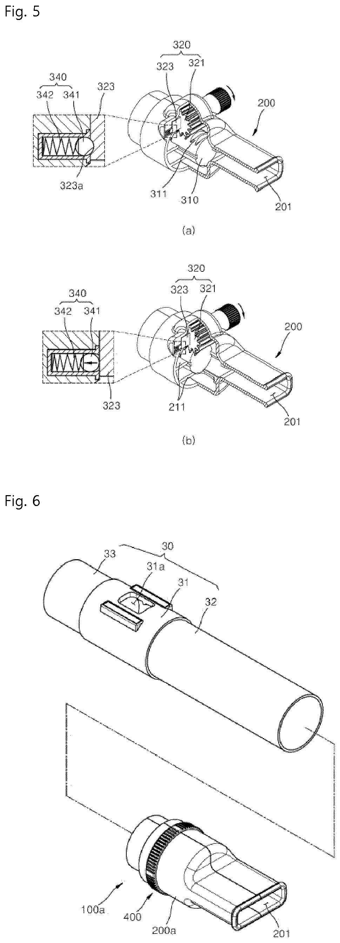

Referring to FIG. 5, the recognition means 340 may include a vibration ball 341 and a support spring 342. Here, the rotation shaft 323 may have a locking groove 323a formed on a side surface thereof in contact with the recognition means 340. The locking groove 323a is spaced apart from the axis center of the rotation shaft 323 by a predetermined distance in the radial direction. Further, one or more locking grooves 323a are radially disposed at a predetermined angle in the circumferential direction of the rotation shaft 323, and the same rotate around the axis center of the rotation shaft 323 when the rotation shaft 323 rotates.

In this case, the vibration ball 341 is in contact with the side surface of the rotation shaft 323 corresponding to the position of the locking groove 323a. When the rotation shaft 323 rotates, the vibration ball 341 moves and vibrates back and forth depending on the position of the locking groove 323a. By such vibration, a user may recognize whether the driving gear 321 operates.

FIGs. 5(a) and 5(b) show movement of the vibration ball 341 according to rotation of the rotation shaft 323. FIG. 5(a) shows a case in which the vibration ball 341 is moved to the right by the locking groove 323a, and FIG. 5(b) shows a case in which the vibration ball 341 comes into contact with the flat side surface of the rotation shaft 323 and is moved to the left.

The support spring 342 serves to elastically support the vibration ball 341 toward the rotation shaft 323 so that the vibration ball 341 comes into close contact with the rotation shaft 323.

Meanwhile, the recognition means 340 may be applied to a known ball plunger, but is not limited thereto. Furthermore, this recognition means 340 includes the vibration ball 341 and the support spring 342, and is configured using the user's touch. In addition to this configuration, it goes without saying that various configurations such as a scale using user's vision are applicable as long as the above-mentioned recognition purpose is achievable.

The recognition means 340 is a means configured to allow a user to recognize a degree of rotation of the rotation lever 330 by himself or herself when the user rotates the rotation lever 330, and the user may recognize rotation of the rotation lever 330 and a degree of pressure control by vibration of the vibration ball 341.

FIGs. 6 to 8 are views showing a respiration control device 100a according to another embodiment of the present invention, and the respiration control device 100a according to another embodiment of the present invention will be described in detail with reference to the drawings.

First, referring to FIG. 6, the respiration control device 100a according to another embodiment of the present invention may include a body part 200a and a pressure controller 400.

The body part 200a is detachably coupled to the above-described mouthpiece 30, and when a user directly puts his or her mouth on the respiration control device 100a to breathe in and out, air by user's respiration may flow into the mouthpiece 30 through an internal flow path.

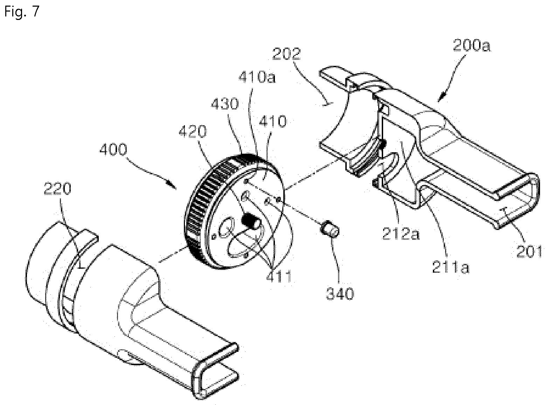

Referring to FIG. 7, the body part 200a may have an inflow part 201 formed on one side and configured to allow air by user's respiration to flow thereinto, an outflow part 202 formed on the other side to be coupled to the mouthpiece 30 and configured to allow the air flowing thereinto to flow out therethrough, and a partition wall 211a formed therein, the partition wall 211a having an inlet part 212a formed to penetrate the same.

The body part 200a may be formed with a fitting portion 220 having the pressure controller 400 coupled thereto. In the drawing, the body part 200a is divided by the fitting portion 220. When the pressure controller 400 is coupled to the fitting portion 220, each of the divided body parts may be coupled to the front and rear surfaces of the pressure controller 400, respectively. In other words, the body part 200a is configured to be divided into a first body part and a second body part by the fitting portion 220. The first body part may be coupled to the front surface of the pressure controller 400, and the second body part may be coupled to the rear surface of the pressure controller 400.

The pressure controller 400 may be coupled to the body part 200a, and the same may include a rotation member 410, a rotation shaft 420, and an operation part 430.

The rotation member 410 is axially coupled to the rotation shaft 420 in a disk shape and is rotatably installed in the body part 200a. Further, the rotation member 410 may have a plurality of pressure control holes 411 formed to pass therethrough and spaced apart from each other in the circumferential direction around the rotation shaft 420. Here, the pressure control holes 411 communicate with the inlet part 212a of the partition wall 211a when the rotation member 410 rotates, and the same allow air introduced from the inlet part 212a to flow into the mouthpiece 30 while passing therethrough.

Meanwhile, the pressure control holes 411 are respectively formed to have different air flow passage areas, and as such pressure of flowing air is controlled by an air passage cross-sectional area of each of the air flow passage areas. In the drawing, the pressure control holes 411 are formed of four different air passage cross-sectional areas so as to control respiration pressure. Here, as the air passage cross-sectional area increases, respiration pressure decreases.

The operation part 430 may be provided on the outer circumferential surface of the rotation member 410 and exposed to the outside of the body part 200a. Accordingly, a user operates the operation part 430 to rotate the rotation member 410 around the rotation shaft. As shown in the drawing, the operation part 430 may have a plurality of protrusions formed thereon and configured to improve the user's grip and facilitate user's operation, but is not limited thereto.

According to the above description, in the pressure controller 400, when a user rotates the rotation member 410 through the operation part 430, pressure is controlled while any one of the pressure control holes 411 having different passage cross-sectional areas communicates with the inlet part 212a.

Hereinafter, the operation of the respiration control device 100a according to the operation of the pressure controller 400 will be described.

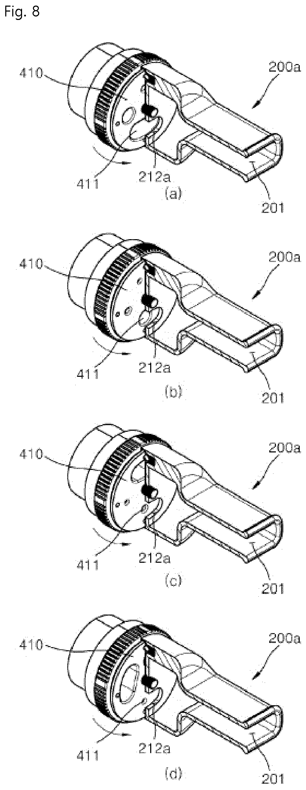

Referring to FIG. 8, the pressure control holes 411 are formed to have different air passage cross-sectional areas as shown in the drawing, and the pressure controller 400 may control respiration pressure according to the air passage cross-sectional area of any one of the pressure control holes 411 communicating with the inlet part 212a.

In detail, first, FIG. 8(a) shows a case in which the air passage cross-sectional area of the pressure control hole 411 communicating with the inlet part 212a is the largest, and respiration pressure is the lowest. Next, FIG. 8(b) shows a case in which the rotation member 410 is rotated by user's operation, and the pressure control hole 411 communicating with the inlet part 212a has a smaller air passage cross-sectional area than that of FIG. 8(a). In this case, respiration pressure may be higher than that of FIG. 8(a). Next, FIG. 8(c) shows a case in which the rotation member 410 is rotated by user's operation, and the pressure control hole 411 communicating with the inlet part 212a has a smaller air passage cross-sectional area than that of FIG. 8(b). In this case, respiration pressure may be higher than that of FIG. 8(b). In addition, FIG. 8(d) shows a case in which the rotation member 410 is rotated by user's operation, and the air passage cross-sectional area of the pressure control hole 411 communicating with the inlet part 212a is the smallest. In this case, respiration pressure is the highest.

According to the above description, the pressure controller 400 is configured so that a user may selectively control respiration pressure by rotating the rotation member 410 through the operation part 430, and respiration pressure may be controlled by varying the size of the air passage cross-sectional area of the pressure control hole 411.

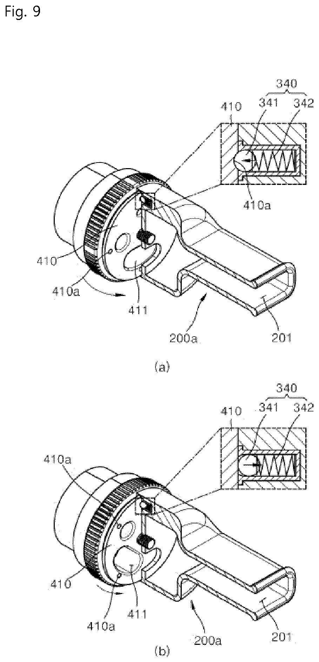

FIG. 9 is a diagram showing the operation of the recognition means 340 provided in the pressure controller 400 described above. Referring to the drawing, the recognition means 340 has substantially the same configuration as that of FIG. 5 including the vibration ball 341 and the support spring 342. Therefore, hereinafter, the operation of the recognition means 340 according to the operation of the pressure controller 400 will be mainly described.

First, in the pressure controller 400, the rotation member 410 may have a locking groove 410a formed on the side surface thereof in contact with the recognition means 340 and disposed at a position corresponding to the recognition means 340. In the drawing, the plurality of locking grooves 410a are radially disposed to be spaced apart from each other in the circumferential direction around the rotation shaft, and the same are rotated by rotation of the rotation member 410.

FIGs. 9(a) and 9(b) show movement of the vibration ball 341 by rotation of the rotation member 410. Referring to the drawings, FIG. 9(a) shows a case in which the vibration ball is moved to the left in the drawing by the locking groove 410a, and FIG. 9(b) shows a case in which the vibration ball 341 is moved to the right by contacting the flat side surface of the rotation member 410.

Meanwhile, the respiration control device 100 may be detachably coupled to the mouthpiece 30, thereby forming a mouthpiece assembly according to an embodiment of the present invention. Here, the mouthpiece assembly according to the embodiment of the present invention may include the above-described respiration control device 100 or 100a, and the mouthpiece 30, detachably coupled to the main body 20 including the respiration measurement module 21, formed to have an air flow path therein, and coupled to the respiration control device 100 or 100a.

Although the present invention has been described with reference to the embodiments shown in the drawings for illustrative purposes, those skilled in the art will appreciate that various modifications and equivalent other embodiments are possible therefrom. Therefore, the true technical protection scope of the present invention should be determined by the technical spirit of the appended claims.

### [Industrial Applicability]

As is apparent from the above description, the present invention may be applied to a respiration measurement device.

## Claims

1. A respiration control device coupled to a mouthpiece of a respiration measurement device and configured to control respiration pressure of a user during user's respiration, the respiration control device comprising:
a body part comprising an inflow part, an outflow part, and an internal flow path, wherein the inflow part is formed on one side of the body part and configured to allow air by the user's respiration to flow thereinto, wherein the outflow part is formed on the other side of the body part, configured to allow the air flowing into the inflow part to flow out therethrough, and coupled to the mouthpiece, and wherein the internal flow path is formed in the body part and configured to allow the inflow part and the outflow part to communicate with each other; and
a pressure controller provided on the internal flow path and configured to control pressure of the air flowing through the internal flow path by an operation of the user,
wherein the pressure controller comprises:
a moving member provided on the internal flow path and configured to reciprocate thereon, wherein the moving member adjusts an air passage cross-section area of the internal flow path by reciprocation thereof and controls the pressure of the air;
a driving part rotatably installed in the body part, coupled to the moving member, and configured to allow the moving member to reciprocate by rotation thereof; and
a rotation lever rotated by the operation of the user and axially coupled to the driving part, wherein the rotation lever rotates the driving part by rotation thereof.

2. The respiration control device according to claim 1, wherein the moving member has a plurality of moving grooves formed on an outer surface thereof in a movement direction thereof, and
wherein the driving part is rotated in conjunction with the rotation of the rotation lever, and comprises a driving gear having screw threads formed on an outer circumferential surface thereof and respectively engaged with the moving grooves, wherein the moving member reciprocates by rotation of the driving gear.

3. The respiration control device according to claim 2, wherein the body part has a control room formed therein, wherein the control room has the pressure controller installed therein, and wherein the control room allows, when the air is introduced from an inlet formed at a front end of the control room in the movement direction of the moving member, the air to flow out through an outlet formed on a side of the moving member, and controls an air passage cross-sectional area of each of the inlet and the outlet by movement of the moving member, and
wherein the inflow part communicates with the inlet of the control room and is formed in a square duct tube shape corresponding to a mouth size of the user.

4. The respiration control device according to claim 3, wherein the body part comprises a partition wall formed therein and configured to support the moving member, the partition wall forming the control room, and
wherein the moving member has a guide part formed on an outer surface thereof and configured to enable the moving member to move along the partition wall.

5. A mouthpiece assembly comprising:
the respiration control device according to any one of claims 1 to 4; and
a mouthpiece detachably coupled to a main body comprising a respiration measurement module, wherein the mouthpiece has an air flow path formed therein and is coupled to the respiration control device.
